(19) Europäisches Patentamt
European Patent Office
Office européen des brevets

(11) Veröffentlichungsnummer : **0 400 383 B1**

# (12) EUROPÄISCHE PATENTSCHRIFT

(45) Veröffentlichungstag der Patentschrift :
**03.03.93 Patentblatt 93/09**

(21) Anmeldenummer : **90108995.3**

(22) Anmeldetag : **12.05.90**

(51) Int. Cl.$^5$ : **C07C 275/26,** C07C 275/28, A61K 6/08, A61K 6/09, C08F 20/00

## (54) Harnstoffgruppen enthaltende (Meth)-Acrylsäure-Derivate von Triisocyanaten und ihre Verwendung.

(30) Priorität : **27.05.89 DE 3917320**

(43) Veröffentlichungstag der Anmeldung :
**05.12.90 Patentblatt 90/49**

(45) Bekanntmachung des Hinweises auf die Patenterteilung :
**03.03.93 Patentblatt 93/09**

(84) Benannte Vertragsstaaten :
**AT BE CH DE DK ES FR GB GR IT LI LU NL SE**

(56) Entgegenhaltungen :
**EP-A- 0 209 365**
**EP-A- 0 266 589**
**DE-A- 2 308 036**

(73) Patentinhaber : **BAYER AG**
**W-5090 Leverkusen 1 Bayerwerk (DE)**

(72) Erfinder : **Müller, Michael, Dr.**
**Richard-Zanders-Strasse 34**
**W-5060 Bergisch Gladbach 2 (DE)**
Erfinder : **Podszun, Wolfgang, Dr.**
**Roggendorfstrasse 55**
**W-5000 Köln 80 (DE)**
Erfinder : **Winkel, Jens, Dr.**
**Letterhausstrasse 1**
**W-5000 Koeln 71 (DE)**

EP 0 400 383 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue Harnstoffgruppen enthaltende Acylsäure- und Methacrylsäure-Derivate von Triisocyanaten, im folgenden (Meth)-Acrylsäure-Derivate genannt, und ihre Herstellung. Die neuen Verbindungen können als Monomere für die Anwendung im Dentalbereich eingesetzt werden.

Es wurden neue Harnstoffgruppen enthaltende (Meth)-Acrylsäure-Derivate von Triisocyanaten der Formel

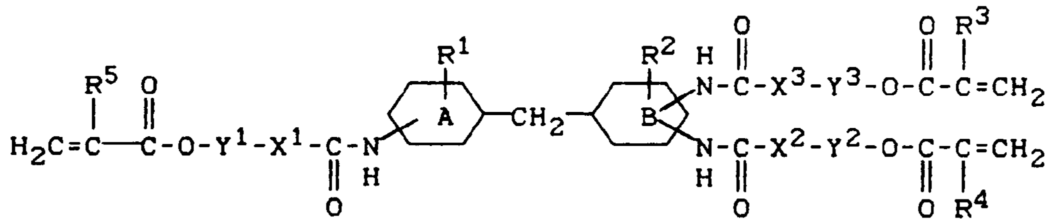

(I)

gefunden, in der

$R^1$ und $R^2$ gleich oder verschieden sind und für Wasserstoff oder einen Niederalkylrest stehen,

$R^3$, $R^4$ und $R^5$ gleich oder verschieden sind und Wasserstoff oder Methyl bedeuten,

$Y^1$ bis $Y^3$ gleich oder verschieden sind und zweiwertige geradkettige oder verzweigte Kohlenwasserstoffreste mit 2 bis 15 Kohlenstoffatomen,die gegebenenfalls 1 bis 3 Sauerstoffbrücken enthalten können und gegebenenfalls durch 1 bis 4 zusätzliche (Meth)-Acryloyl-oxy-Reste substituiert sein können, bedeuten,

$X^1$ bis $X^3$ gleich oder verschieden sind und $-NR^1-$ oder -O- bedeuten, wobei mindestens einer der Reste $X^1$ bis $X^3$ $-NR^1-$ bedeutet und $R^1$ die oben beschriebene Bedeutung hat und die Ringe

A und B gleich oder verschieden sind und aromatisch oder gesättigt sein können.

Die (Meth)-Acrylsaure-Derivate können als reine Isomere oder als Isomerengemisch vorliegen. Für die erfindungsgemäße Verwendung der (Meth)-Acrylsäure-Derivate in Dentalmaterialien ist es besonders vorteilhaft, die Isomerengemische einzusetzen, da sie eine niedrigere Viskosität als die isomerenreinen Verbindungen besitzen.

Im Rahmen der vorliegenden Erfindung können die Substituenten im allgemeinen folgende Bedeutung haben.

Niederalkyl kann einen geradkettigen oder verzweigten Kohlenwasserstoffrest mit 1 bis etwa 6 Kohlenstoffatomen, bevorzugt 1 bis 4 Kohlenstoffatomen bedeuten. Beispielsweise seien die folgenden Niederalkylreste genannt: Methyl, Ethyl, Propyl, Isopropyl, Butyl, Isobutyl, Pentyl, Isopentyl, Hexyl und Isohexyl.

Zweiwertige Kohlenwasserstoffreste $Y^1$ bis $Y^3$ können geradkettige oder verzweigte aliphatische Kohlenwasserstoffreste mit 2 bis 15 Kohlenstoffatomen, bevorzugt 2 bis 10 Kohlenstoffatomen bedeuten. Die Reste $Y^1$ bis $Y^3$ können gegebenenfalls jeweils 1 bis 3 Sauerstoffbrücken, bevorzugt 1 oder 2 Sauerstoffbrücken enthalten. Es ist auch möglich, daß die Reste $Y^1$ bis $Y^3$ durch 1 bis 4, bevorzugt 1 oder 2 (Meth)-Acryloyloxyreste, substituiert sind. Beispielsweise seien die folgenden Reste genannt:

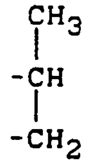 , 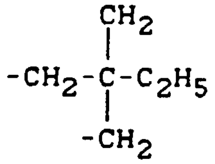 , 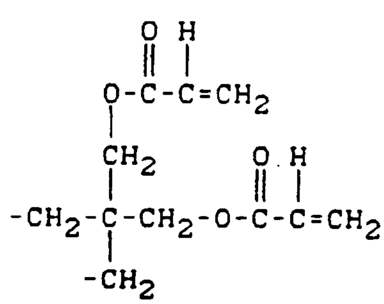 ,

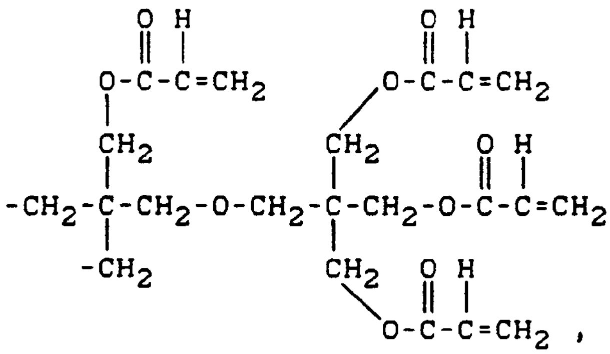

,

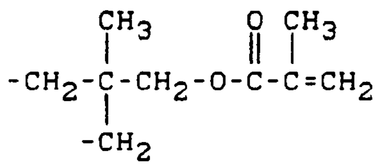

,

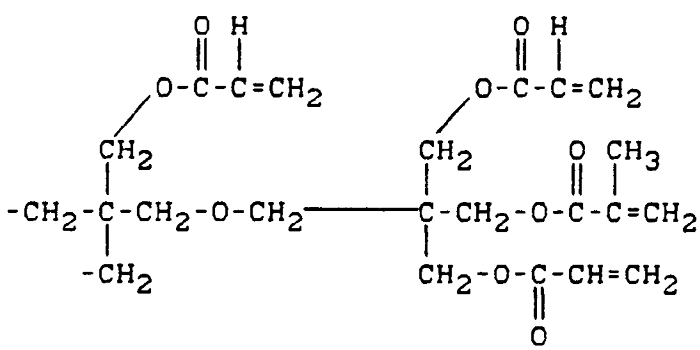

,

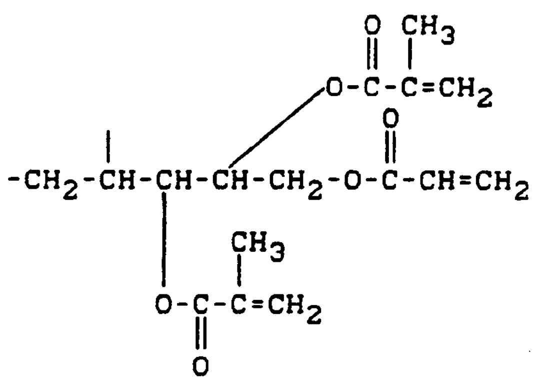

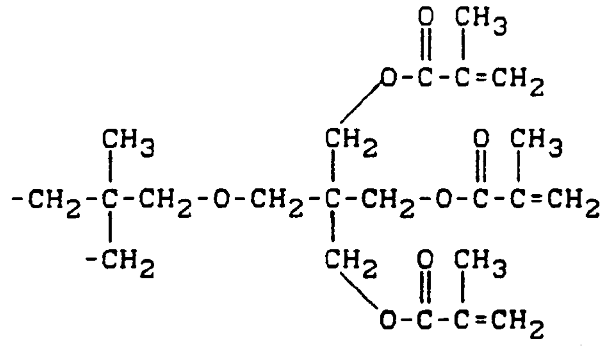
,

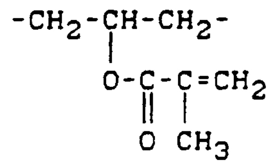
,
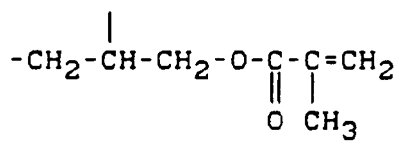
,

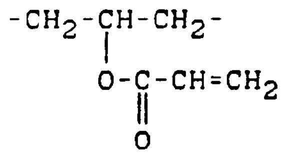
,
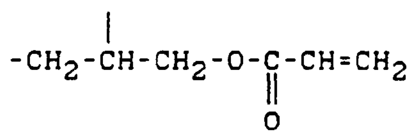
.

Der Ring A steht für einen Benzolkern oder einen Cyclohexanrest, der zwei, bzw. drei Substituenten enthält. Der Ring B steht für einen Benzolkern oder einen Cyclohexanrest, der drei bzw. vier Substituenten enthält.

Die neuen (Meth)-Acrylsäure-Derivate sind farblos, schwerflüchtig und ergeben nach der Polymerisation transparente Kunststoffe mit hoher Verschleißfestigkeit.

Sie lassen sich besonders gut in Dentalmaterialien, wie Zahnfüllmassen und Beschicbtungsmitteln, verwenden. Die so erhaltenen Materialien zeichnen sich durch eine überraschend große Widerstandsfähigkeit gegenüber physikalischer und chemischer Beanspruchung aus. In besonderem Maße sind Härte und Bruchfestigkeit gegenüber üblichen, zu diesem Zweck eingesetzten Materialien, verbessert.

In der DE-OS 3 636 189 werden (Meth)-Acrylsäure-Derivate von Triisocyanaten beschrieben, die als Bindeglied zwischen (Meth)-acrylat- und Triisocyanat-Einheit ausschließlich Urethangruppen enthalten. Diese Verbindungen zeigen im wesentlichen drei entscheidende Nachteile, die ihre Verwendung erschweren. Sie sind so hochviskos, daß sie mit großen Mengen an Comonomeren abgemischt werden müssen, die das Eigenschaftniveau der ausgehärteten Polymerisate verschlechtern. Ihre Darstellung erfordert lange Reaktionszeiten, die über eine Tag hinausgehen. Außerdem erfolgt ihre Darstellung in einem niedrig siedenden, inerten Lösungsmittel, das in einem zusätzlichen Verfahrensschritt gegen Comonomere ausgetauscht werden muß.

Alle drei Probleme ließen sich mit Hilfe der erfindungsgemäßen Harnstoffgruppen enthaltenden (Meth)-Acrylsäure-Derivate von Triisocyanaten lösen, indem sich diese direkt im Gemisch mit geeigneten Comonomeren, in deutlich kürzeren Reaktionszeiten, in Form niederviskoser Öle darstellen ließen, was aufgrund der üblicherweise erhöhten Kristallisationsneigung von Harnstoff-Derivaten überraschte.

Bevorzugte erfindungsgemäße (Meth)-Acrylsäure-Derivate sind Verbindungen der Formel I

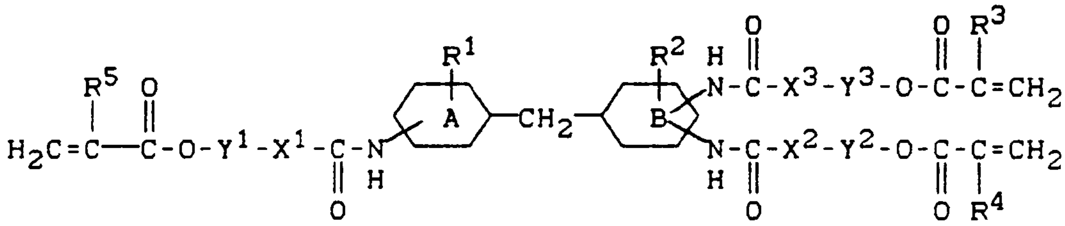

(I)

in der

$R^1$ für Wasserstoff steht,

$R^2$ für Wasserstoff oder einen Alkylrest mit 1 bis 4 Kohlenstoffatomen steht,

$R^3$, $R^4$ und $R^5$ gleich oder verschieden sind und Wasserstoff oder Methyl bedeuten,

$Y^1$ bis $Y^3$ gleich oder verschieden sind und zweiwertige geradkettige oder verzweigte aliphatische Kohlenwasserstoffreste mit 2 bis 10 Kohlenstoffatomen, die gegebenenfalls 1 oder 2 Sauerstoffbrücken enthalten können und gegebenenfalls durch 1 bis 2 zusätzliche (Meth)-Acryloyloxyreste substituiert sein können, bedeuten,

$X^1$ für -O- steht,

$X^2$ und $X^3$ die Bedeutung von $-NR^1-$ haben,

der Ring B aromatisch oder gesättigt ist und

der Ring A gesättigt ist.

Insbesondere bevorzugt sind (Meth)-Acrylsaure-Derivate der Formel:

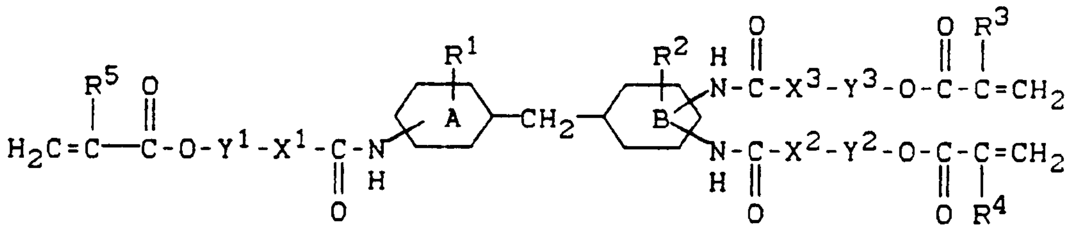

(I)

in der

$R^1$ für Wasserstoff steht,

$R^2$ für Wasserstoff oder Methyl steht,

$R^3$, $R^4$ und $R^5$ gleich oder verschieden sind und Wasserstoff oder Methyl bedeuten,

$Y^1$ bis $Y^3$ gleich oder verschieden sind und zweiwertige geradkettige oder verzweigte aliphatische Kohlenwasserstoffreste mit 2 bis 10 Kohlenstoffatomen, die gegebenenfalls 1 oder 2 Sauerstoffbrücken enthalten können und gegebenenfalls durch 1 oder 2 zusätzliche (Meth)-Acryloyloxyreste substituiert sein können, bedeuten,

$X^1$ und $X^2$ für -O- stehen,

$X^3$ die Bedeutung von $NR^1$ hat,

und die Ringe A und B gesättigt sind.

Beispielweise seien die folgenden (Meth)-Acrylsäure-Derivate genannt:

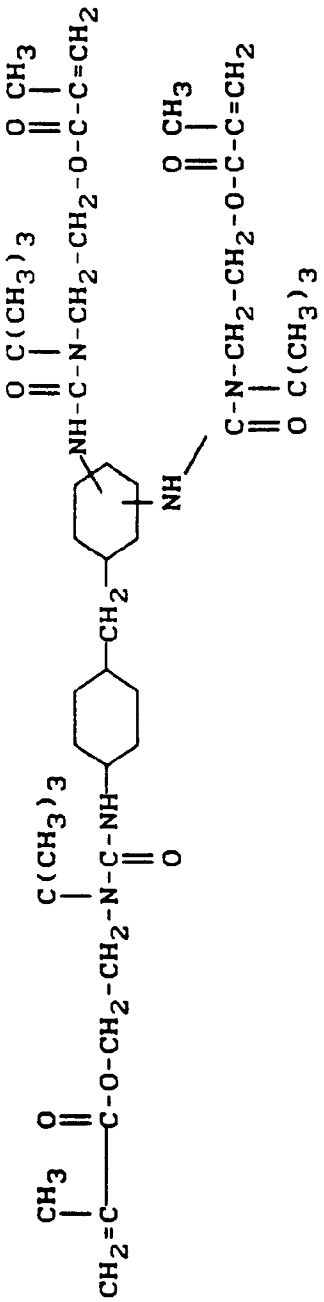

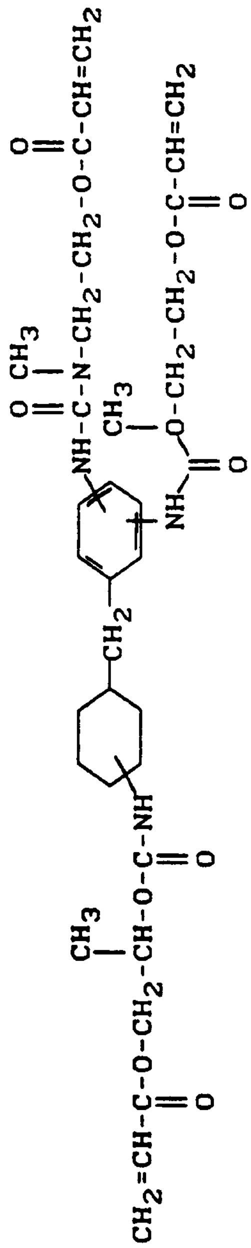

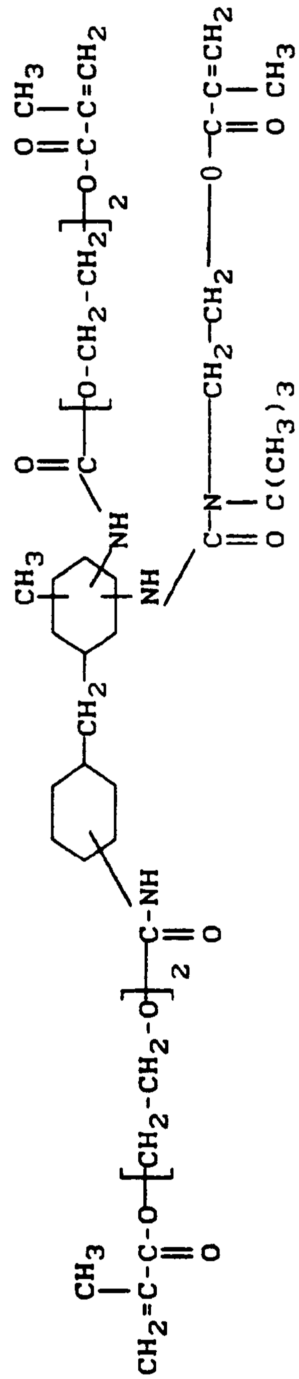

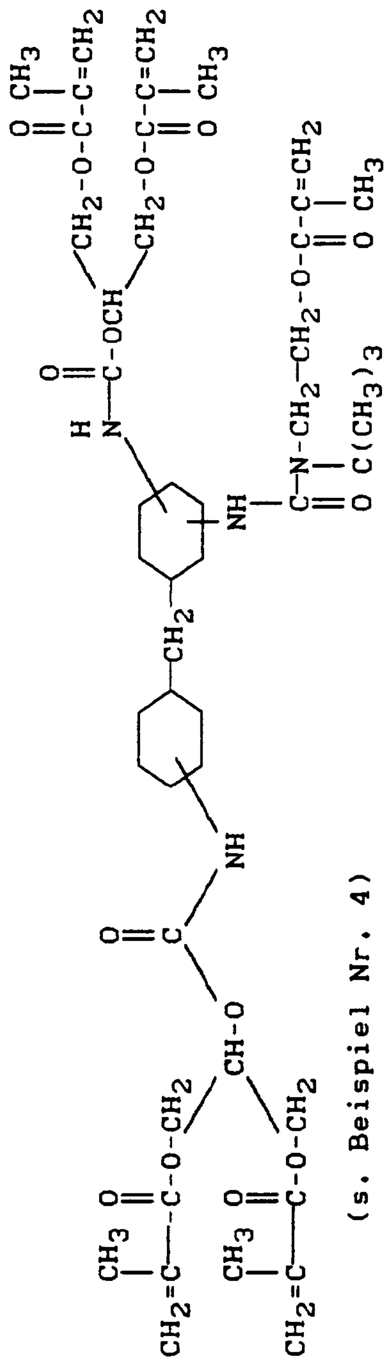

(s. Beispiel Nr. 4)

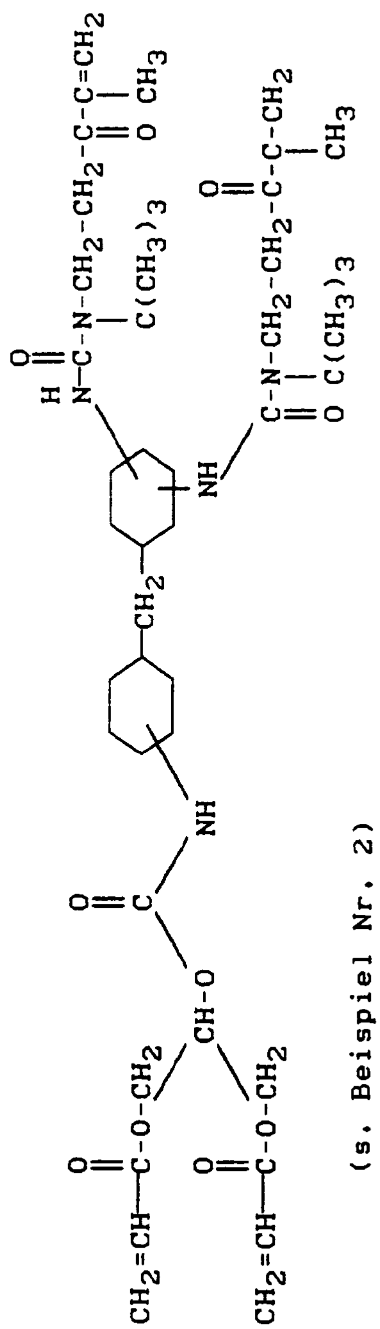

(s. Beispiel Nr. 2)

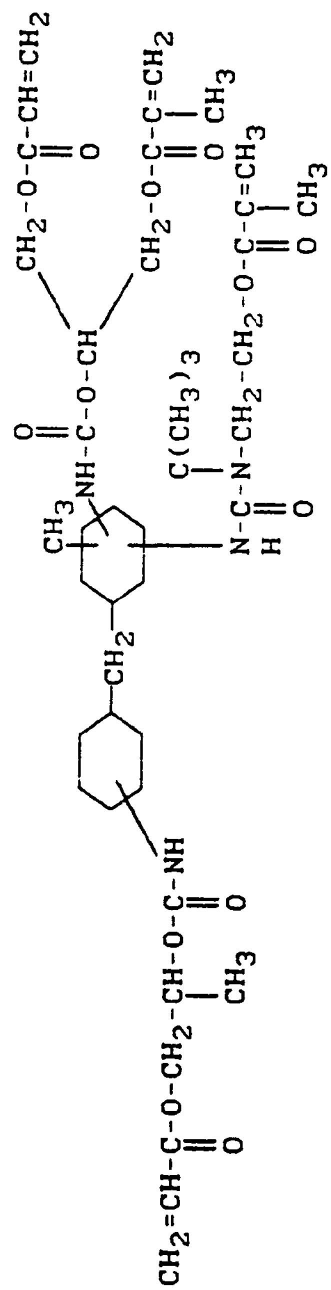

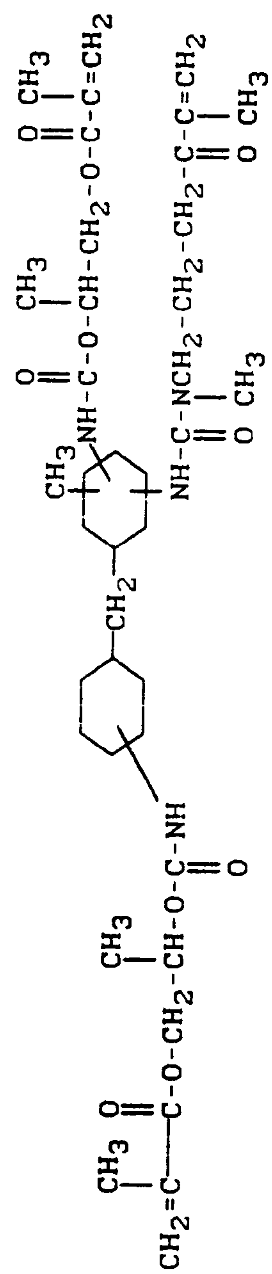

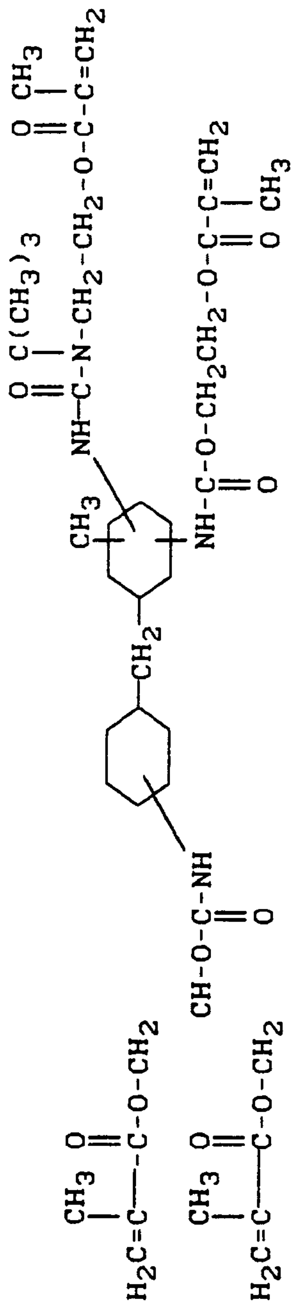

(s. Beispiel Nr. 1)

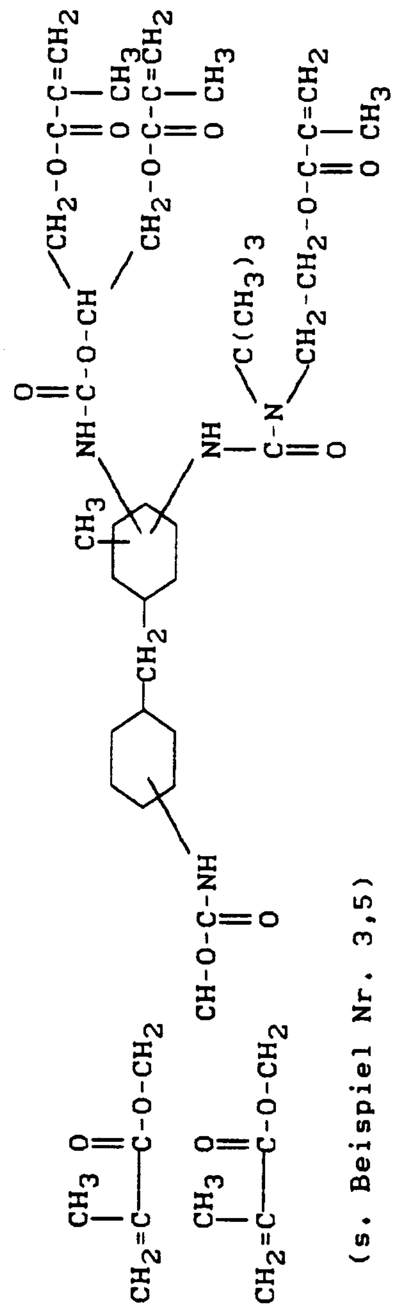

(s. Beispiel Nr. 3,5)

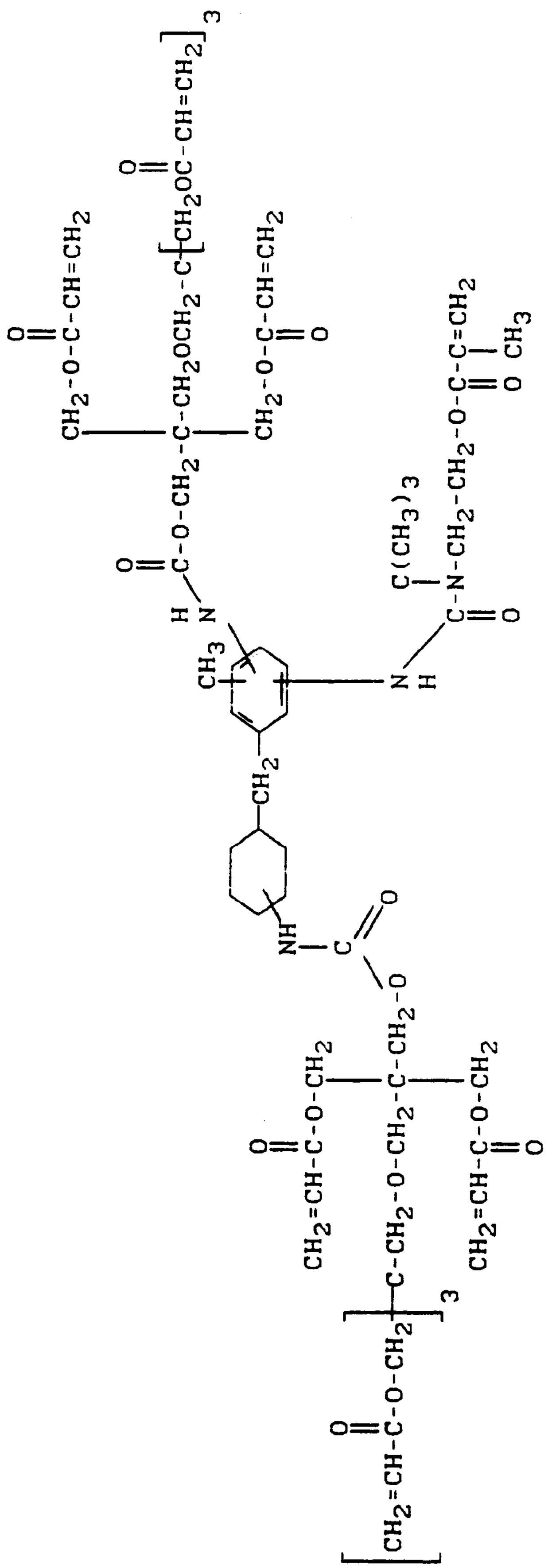

Es wurde auch ein Verfahren zur Herstellung der erfindungsgemäßen (Meth)-Acrylsäure-Derivate gefunden, das dadurch gekennzeichnet ist, daß man ein Triisocyanat der Formel II

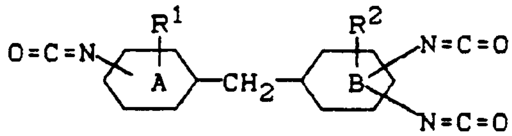

(II)

in der

$R^1$ und $R^2$ gleich oder verschieden sind und für Wasserstoff oder einen Niederalkylrest stehen, und die Ringe

A und B gleich oder verschieden sind und aromatisch oder gesättigt sein können,

mit Aminoalkyl-(Meth)-Acrylsäureester der Formel

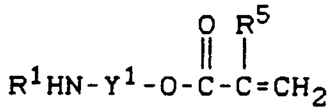

(III)

und/oder

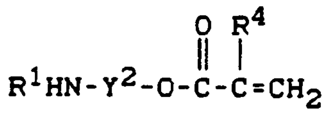

(IV)

und/oder

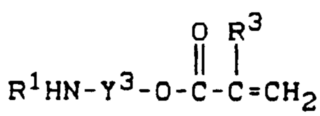

(V)

und gegebenenfalls mit Hydroxyalkyl-(Meth)-Acrylsäureestern der Formel

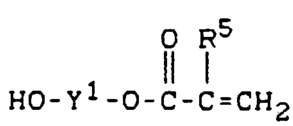

(VI)

und/oder

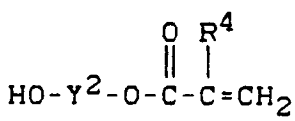

(VII)

und/oder

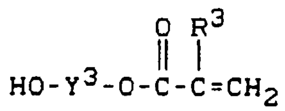

(VIII)

in denen

$R^3$, $R^4$ und $R^5$ gleich oder verschieden sind und Wasserstoff oder Methyl bedeuten,

$Y^1$ bis $Y^3$ gleich oder verschieden sind und zweiwertige geradkettige oder verzweigte aliphatische Kohlenwasserstoffreste mit 2 bis 15 Kohlenstoffatomen, die gegebenenfalls 1 bis 3 Sauerstoffbrücken enthalten können und gegebenenfalls durch 1 bis 4 zusätzliche (Meth)-Acryloyloxyreste substituiert sein können, bedeuten, und

$R^1$ die oben beschriebene Bedeutung hat,

umsetzt.

Triisocyanate der Formel II sind bekannt (DE-Al 3417684, DE-Al 3417683) und können durch Phosgenierung der entsprechenden Triaminoverbindungen erhalten werden.

Hydroxyalkyl-(Meth)-Acrylsäureester der Formel VI bis VIII und Aminoalkyl-(Meth)-Acrylsäureester der Formel III bis V sind handelsüblich oder können in bekannter Weise durch partielle Veresterung der entsprechenden Polyole bzw. Veresterung von Alkanolaminen gegebenenfalls unter Verwendung von Schutzgruppen für die Aminofunktion hergestellt werden.

Das erfindungsgemäße Verfahren wird im allgemeinen so durchgeführt, daß man, bezogen auf jede Isocyanatgruppe des Triisocyanats (II) 0 bis 0,8 Äquivalente eines Hydroxyalkyl-(Meth)-Acrylsäureesters der Formel VI, VII oder VIII und 0,2 bis 1,1 Äquivalente eines Aminalkyl-(Meth)-Acrylsäureesters der Formel III, IV oder V oder Gemische der Verbindungen III bis VIII einsetzt, wobei die Summe aller Hydroxyläquivalente pro Isocyanatgruppe 0 bis 0,8 und die Summe aller Aminoäquivalente pro Isocyanatgruppe 0,2 bis 1,1 in der Weise ergeben soll, daß die Summe aus Hydroxyl- und Aminoäquivalenten pro Isocyanatgruppe 0,9 bis 1,1 ergibt.

Bevorzugt werden Verfahren, in denen bezogen auf jede Isocyanatgruppe des Triisocyanats (II) je 0,3 bis 0,7 Äquivalente eines Hydroxyalkyl-(Meth)-Acrylsäureesters der Formel VI, VII oder VIII und eines Aminoalkyl-(Meth)-Acrylsäureesters der Formel III, IV, oder V oder Gemische der Verbindungen III bis VIII eingesetzt werden, wobei die Summe aller Hydroxyläquivalente und die Summe aller Aminoäquivalente jeweils 0,3 bis 0,7 in der Weise ergeben soll, daß die Summe aus Hydroxyl- und Aminoäquivalenten pro Isocyanatgruppe 1,0 bis 1,05 ergibt.

Das erfindungsgemäße Verfahren wird unter Wasserausschluß in einem inerten Lösungsmittel durchgeführt. Beispielsweise seien Chloroform, Tetrahydrofuran, Aceton, Dioxan, Dichlormethan, Toluol und Acetonitril genannt. Bevorzugte Lösungsmittel sind Chloroform, Toluol, Aceton und Dichlormethan.

Insbesondere kann das erfindungsgemäße Verfahren auch in einem Reaktivverdünner als Lösungsmittel erfolgen, der selbst (Meth)acrylatgruppen enthält und deshalb nach der Reaktion nicht entfernt werden muß, da er mit den erfindungsgemäßen Verbindungen copolymerisierbar ist. Das erhaltene Monomergemisch ist so direkt zur Herstellung von Dentalmaterialien geeignet. Geeignete Reaktivverdünner sind Mono- und Dimethacrylate von zweiwertigen Alkoholen wie Alkandiolen oder Ethylenglykolen und Propylenglykolen mit zwei bis zwölf Kohlenstoffatomen. Besonders geeignet sind Hexandioldimethacrylat und Triethylenglykoldimethacrylat, Ethylenglykoldimethacrylat und Neopentylglykoldimethacrylat.

Das erfindungsgemäße Verfahren wird im allgemeinen im Temperaturbereich von 20 bis 100°C, vorzugsweise von 30 bis 70°C, durchgeführt.

Das erfindungsgemäße Verfahren wird im allgemeinen bei Normaldruck durchgeführt. Es ist jedoch auch möglich, das Verfahren im Druckbereich von 1 bis 15 bar durchzuführen.

Bei der erfindungsgemäßen Umsetzung des Isocyanates (II) wird vorzugsweise unter Wasserausschluß gearbeitet (vorzugsewise unter 0,1 % Wasser).

Zur Beschleunigung der Umsetzung werden vorzugsweise zinnhaltige Katalysatoren wie Dibutylzinndilaurat, Zinn(II)-octoat oder Dibutylzinndimethoxid verwendet.

Es ist auch möglich, als Katalysatoren Verbindungen mit tertiären Aminogruppen oder Titanverbindungen einzusetzen. Beispielsweise seien die folgenden Katalysatoren genannt: Diazabicyclo[2.2.2]octan, Triethylamin, N-Methylpiperidin, Tetrabutoxy-titan (Ullmann, Encyclopädie der technischen Chemie, Bd. 19, S. 306 (1981)).

Im allgemeinen wird der Katalysator in einer Menge von 0,01 bis 2,5 Gew.-%, bevorzugt von 0,1 bis 1,5 Gew.-%, bezogen auf die Gesamtmenge der Reaktanden eingesetzt.

Die Umsetzung wird im allgemeinen in Gegenwart von 0,01 bis 0,2 Gew.-% eines Polymerisationsinhibitors, beispielsweise 2,6-Di-tert.-butyl-4-methylphenol durchgeführt.

Das erfindungsgemäße Verfahren kann beispielsweise wie folgt durchgeführt werden:

Der Hydroxylalkyl-(Meth)-Acrylsäureester (VI-VIII) wird in einem Lösungsmittel gelöst und unter Rühren zuerst mit dem Katalysator und dann mit dem Triisocyanat (II) versetzt. Nach Umsatz aller Hydroxylfunktionen (IR-Kontrolle, NCO-Titration) wird der Aminoalkyl-(Meth)-Acrylsäureester (III-V) zugetropft. Nach vollständiger Umsetzung der Isocyanatgruppen werden die Reaktionsprodukte durch Entfernen des Lösungsmittels isoliert. Eine vorherige Reinigung mit Hilfe von Absorbentien, beispielsweise Aktivkohle, Bleicherde, Kieselgel oder Aluminiumoxid ist selbstverständlich auch möglich.

Die erfindungsgemäßen (Meth)-Acrylsäure-Derivate von Triisocyanaten können als Monomere zur Herstellung von polymeren Werkstoffen verwendet werden. Die Polymerisation kann in an sich bekannter Weise durch radikalische Initiierung durchgeführt werden und ergibt Polymerisate, die eine hohe Vernetzungsdichte aufweisen.

Die erfindungsgemäßen (Meth)-Acrylsäurederivate von Triisocyanaten können insbesondere als Monomere für Dentalmaterialien verwendet werden. Als Dentalmaterialien seien beispielsweise Füllungsmaterialien für Zähne, Beschichtungsmittel für Zähne und Komponenten für die Herstellung von Zahnersatz genannt. Je nach Anwendungsgebiet können Dentalmaterialien weitere Hilfsstoffe enthalten.

Für die Anwendung als Monomere für Zahnfüllmassen oder Beschichtungsmittel (Zahnlacke) im Dentalbereich können die erfindungsgemäßen (Meth)-Acrylsäure-Derivate von Triisocyanaten mit an sich bekannten Comonomeren gemischt werden. So kann beispielsweise die Viskosität dem Verwendungszweck angepaßt werden. Diese Monomermischungen haben im allgemeinen eine Viskosität im Bereich von 60 bis 20.000 mPa.s.

Beispielsweise seien die folgenden Comonomere genannt:

Triethylenglykoldimethacrylat, Tetraethylenglykoldimethacrylat, 1,12-Dodecandioldimethacrylat, 1,6-Hexandioldimethacrylat, Diethylenglykoldimethacrylat, 2,2-Bis[p-(2′-hydroxy-3′-methacryloyloxypropoxy) phenyl]propan, 2,2-Bis[p-(2′-methacryloyloxyethoxy)phenyl]propan. Von Vorteil sind auch Comonomere mit Urethangruppen, z. B. die bekannten Umsetzungsprodukte von 1 Mol eines Diisocyanats, z. B. Hexamethylendiisocyanat, Trimethylhexamethylendiisocyanat, Isophorondiisocyanat, mit 2 Molen eines Hydroxyalkyl-(meth)acrylsäureesters, z. B. Glycerindimethacrylat, 2-Hydroxypropylacrylat usw..

Weitere Beispiele für Comonomere sind:

Trimethylolpropan-tri(meth)-acrylat, Bis-(Meth)acryloyloxyethoxymethyl)-tricyclo[5.2.1.$0^{2.6}$]decan (gemäß DE-A 2 931 925 und 2 931 926), 1,3-Di((meth)acryloyloxypropyl)-1,1,3,3-tetramethyl-disiloxan, 1,3-Bis(3-(meth)-acryloyloxyethylcarbamoyloxy-propyl)-1,1,3,3-tetramethyl-disiloxan. Insbesondere werden Comonomere bevorzugt, die bei 13 mbar einen Siedepunkt über 100°C besitzen.

Im Rahmen der vorliegenden Erfindung ist es ebenfalls bevorzugt, Mischungen verschiedener erfindungsgemäßer (Meth)-Acrylsäure-Derivate einzusetzen.

Der Anteil der erfindungsgemäßen Harnstoffgruppen enthaltenden (Meth)Acrylsäure-Derivate von Triisocyanaten in den Monomermischungen beträgt im allgemeinen 10 bis 90 Gew.-%, vorzugsweise 20 bis 75 Gew.-%.

Es ist auch möglich, Monomermischungen einzusetzen, die mehrere Comonomere enthalten.

Die erfindungsgemäßen Harnstoffgruppen enthaltenden (Meth)-Acrylsäure-Derivate von Triisocyanaten lassen sich, gegebenenfalls in Mischung mit den genannten Monomeren, mit an sich bekannten Methoden zu vernetzten Polymerisaten aushärten (G.M. Brauer, H. Argentar, Am. Chem. Soc., Symp. Ser. 212, S. 359-371 (1983)). Für die sogenannte Redoxpolymerisation ist ein System aus einer peroxidischen Verbindung und einem Reduktionsmittel, beispielsweise auf Basis tertiärer aromatischer Amine, geeignet. Beispiele für Peroxide sind:

Dibenzoylperoxid, Dilauroylperoxid und Di-4-Chlorbenzoylperoxid.

Als tertiäre aromatische Amine seien beispielsweise N,N-Dimethyl-p-toluidin, Bis-(2-hydroxyethyl)-p-toluidin, Bis(2-hydroxyethyl)-3,5-dimethylanilin und das in der DE-A 2 759 239 beschriebene N-Methyl-N-(2-methyl-carbamoyloxypropyl)-3,5-dimethylanilin genannt.

Die Konzentration des Peroxids bzw. des Amins wird vorteilhaft so gewählt, daß sie 0,1 bis 5 Gew.-%, bevorzugt 0,5 bis 3 Gew.-%, bezogen auf die Monomermischung beträgt. Die Peroxid- bzw. aminhaltige Monomermischungen werden bis zur Anwendung getrennt gelagert.

Die erfindungsgemäßen Monomeren können auch durch Bestrahlung mit UV-Licht oder sichtbarem Licht (beispielsweise im Wellenlängenbereich von 230 bis 650 nm) zur Polymerisation gebracht werden. Als Initiatoren für die fotoinitiierte Polymerisation eignen sich beispielsweise Benzil, Benzildimethylketal, Benzoinmonoalkylether, Benzophenon, p-Methoxybenzophenon, Fluorenon, Thioxanthon, Phenanthrenchinon und 2,3-Bornandion (Campherchinon), gegebenenfalls in Gegenwart von synergistisch wirkenden Fotoaktivatoren, wie N,N-Dimethylaminoethylmethacrylat, Triethanolamin, 4-N,N-Dimethyl-aminobenzolsulfonsäurebisallylamid.

Die Durchführung des Fotopolymerisationsverfahrens ist beispielsweise in der DE-A 3 135 115 beschrieben.

Neben den oben beschriebenen Initiatoren können den erfindungsgemäßen (Meth)-Acrylsäureestern an sich für diesen Einsatzzweck bekannte Lichtschutzmittel und Polymerisations-Inhibitoren zugesetzt werden.

Das Lichtschutzmittel und der Polymerisations-Inhibitor werden jeweils im allgemeinen in einer Menge von 0,01 bis 0,50 Gew.-Teilen, bezogen auf 100 Gew.-Teile der Monomermischung eingesetzt. Die Monomermischungen können ohne Zusatz von Füllstoffen als Beschichtungsmittel für Zähne (Zahnlacke) eingesetzt werden. Nach der Polymerisation erhält man einen kratzfesten Überzug auf dem Substrat.

Bei der Verwendung als Zahnfüllmassen setzt man den erhaltenen Monomermischungen im allgemeinen Füllstoffe zu. Um einen hohen Füllgrad erreichen zu können, sind Monomermischungen, die eine Viskosität im Bereich von 60 bis 20.000 mPa.s besitzen, besonders vorteilhaft. Den die erfindungsgemäßen Verbindungen der Formel I enthaltenden Monomermischungen können vorzugsweise anorganische Füllstoffe zugemischt werden. Beispielsweise seien Bergkristall, Quarzit, Kristobalit, Quarzglas, hochdisperse Kieselsäure, Aluminiumoxid und Glaskeramiken, beispielsweise Lanthan und Zirkon enthaltende Glaskeramiken (DE-A 2 347 591) genannt.

Die anorganischen Füllstoffe werden zur Verbesserung des Verbundes zur Polymermatrix des Polymethacrylats vorzugsweise mit einem Haftvermittler vorbehandelt. Die Haftvermittlung kann beispielsweise durch eine Behandlung mit Organosiliciumverbindungen erreicht werden (E.P. Plueddemann, Progress in Organic Coatings, 11, 297 bis 308 (1983)). Bevorzugt wird 3-Methacryloyloxypropyltrimethoxysilan eingesetzt.

Die Füllstoffe für die erfindungsgemäßen Zahnfüllmassen weisen im allgemeinen einen mittleren Teilchendurchmesser von 0,01 bis 100 µm, vorzugsweise von 0,05 bis 50 µm auf, besonders bevorzugt 0,05 bis 5 µm. Es kann auch vorteilhaft sein, mehrere Füllstoffe nebeneinander einzusetzen, die einen voneinander verschiedenen Teilchendurchmesser und verschiedenen Silanisierungsgrad besitzen.

Der Füllstoffanteil in den Zahnfüllmassen beträgt im allgemeinen 5 bis 85 Gew.-%, vorzugsweise 50 bis 80 Gew.-%.

Für die Herstellung der Zahnfüllmassen werden die Komponenten unter Verwendung handelsüblicher Knetmaschinen verarbeitet.

Der Anteil der erfindungsgemäßen (Meth)-Acrylsäure-Derivate in den Füllmassen beträgt im allgemeinen 5 bis 90 Gew.-%, vorzugsweise 10 bis 60 Gew.-%, bezogen auf die Füllmasse. Die Aushärtung der Zahnfüllmassen zu einem Formkörper erfolgt in der Kavität des Zahnes mit den oben genannten Methoden. Aufgrund der hohen Verschleißfestigkeit der erhaltenen Zahn-Füllung sind Zahnfüllmassen, die die erfindungsgemäßen Verbindungen in polymerisierter Form enthalten, insbesondere zur Anwendung im Seitenzahnbereich geeignet.

Die erfindungsgemäßen (Meth)-Acrylsäure-Derivate von Triisocyanaten können auch als Komponenten bei der Herstellung von Zahnersatz eingesetzt werden.

Dabei werden die erfindungsgemäßen Monomeren mit den üblicherweise verwendeten, an sich bekannten Bestandteilen kombiniert. Vorzugsweise werden die Monomeren im Gemisch mit Alkylmethacrylaten, wie Methylmethacrylat eingesetzt. Es können auch zusätzlich an sich bekannte Perlpolymerisate zugesetzt werden. Zur Einstellung der Zahnfarbe können bekannte anorganische und organische Farbpigmente und Trübungsmittel zugesetzt werden. Auch die Anwendung von Stabilisatoren und Lichtschutzmitteln ist möglich.

Die Kunststoffzähne werden durch radikalische Polymerisation der Dentalmassen unter Formgebung hergestellt. Die Verarbeitung ist sowohl nach Injektionsverfahren als auch Prägeverfahren möglich und erfolgt im allgemeinen nach den üblichen Herstellungsmethoden für Zähne auf Basis von Poly(methylmethacrylat), z. B. durch thermische Polymerisation unter Verwendung von an sich bekannten Polymerisationsinitiatoren, beispielsweise auf Basis von Peroxiden und Azoverbindungen, wie Dibenzoylperoxid, Dilauroylperoxid, Cyclohexylpercarbonat, Azoisobuttersäuredinitril. Gut geeignet sind auch Mischungen von Polymerisationsinitiatoren mit unterschiedlichen Halbwertzeichen bezüglich ihres Zerfalls.

Beispiel 1-5: Herstellung von Addukten aus Triisocyanaten, Hydroxyalkyl-(Meth)-Acrylsäureestern und Aminoalkyl-(Meth)-Acrylsäureestern

Im folgenden benutzte Abkürzungen:

| | |
|---|---|
| GDMA: | "Glycerindimethacrylat" (Isomerengemisch aus 1,3- und 1,2-Bis-methacryloyloxy-propanol) |
| HDMA: | "Hexandioldimethacrylat" (1,6-Bis-methacryloyloxyhexan) |
| HEMA: | "2-Hydroxyethylmethacrylat" (2-Methacryloyloxyethanol) |
| BAEMA: | "N-tert.Butylaminoethylmethacrylat" (N-tert.-Butyl-N-2-methacryloyloxyethyl-amin) |
| Triisocyanat: | Triisocyanato-dicyclohexylmethan (Isomerengemisch mit einem NCO-Gehalt von 41,5 |

| | |
|---|---|
| | Gew.-%) |
| M-Triisocyanat: | Triisocyanato-cyclohexyl-methylcyclohexyl-methan (Isomerengemisch mit einem NCO-Gehalt von 38,2 Gew.-%) |
| Stabilisator: | 2,6-Di-tert.butyl-4-methylphenol |
| Katalysator: | Zinn(II)-octoat. |

Allgemeine Arbeitsvorschrift zur Darstellung der erfindungsgemäßen (Meth)-Acrylsäure-Derivate:

Zu einem bei 30°C gerührten Gemisch aus HDMA, GDMA, Stabilisator und Katalysator wird der 1. Zulauf (Triisocyanat, M-Triisocyanat) getropft, auf 55°C erwärmt und bei dieser Temperatur bis zum vollständigen Verbrauch des GDMAs gerührt. Nun wird der einen weiteren Hydroxyalkyl-(Meth)-Acrylsäureester enthaltende 2. Zulauf (z.B. HEMA) zugegeben und erneut so lange bei unveränderter Temperatur gerührt bis auch diese Alkoholkomponente vollständig abreagiert ist. Dann wird der einen Aminoalkyl-(Meth)-Acrylsäureester enthaltende 3. Zulauf (z.B. BAEMA) zugetropft und bei 55°C bis zum vollständigen Verbrauch aller Isocyanatgruppen gerührt. Man erhält ein farbloses Monomerengemisch, das direkt für die Herstellung von Dentalmaterialien eingesetzt werden kann.

Tabelle 1:

Einsatzmengen gemäß der allgemeinen Arbeitsvorschrift (- alle Angaben in g (mol))

| | 1 | 2 | 3 | 4 |
|---|---|---|---|---|
| Vorlage: | | | | |
| HDMA | 90.19 (0.355) | 96.14 (0.355) | 100.0 (0.393) | 191.39 (0.735) |
| GDMA | 54.73 (0.240) | 54.73 (0.240) | 109.45 (0.480) | 109.45 (0.480) |
| Stabilisator | 0.36 (0.002) | 0.38 (0.002) | 0.26 (0.001) | 0.37 (0.002) |
| Katalysator | 0.12 | 0.12 | 0.12 | 0.12 |
| Zulauf 1: | | | | |
| Triisocyanat | | | | 72.91 (0.720NCO) |
| M-Triisocyanat | 79.35 (0.721NCO) | 79.35 (0.721NCO) | 79.35 (0.721NCO) | |
| Zulauf 2: | | | | |
| HEMA | 31.25 (0.240) | - | - | - |
| Zulauf 3: | | | | |
| BAEMA | 45.12 (0.243) | 90.24 (0.487) | 45.12 (0.243) | 45.12 (0.245) |

Beispiel 5

Anstelle des Reaktivverdünners HDMA werden 150 ml Chloroform eingesetzt und ansonsten entsprechend Beispiel 3 verfahren.

Nach beendeter Reaktion wird das Chloroform abgezogen und das erfindungsgemäße Addukt aus M-Triisocyanat, GDMA und BAEMA in Form eines farblosen Öles erhalten.

IR (Film): γ=3360, 2920, 1720, 1640, 1519, 1450, 1320, 1298, 1158, 1007, 942, 812, 752 $cm^{-1}$.

$^1$H-NMR ($CDCl_3$, 360 MHz): δ = 1,38 (s, 3H, Cyclohexyl-$CH_3$), 0,9-1,6 (m, 20H, CH- und $CH_2$ der Dicyclohexylmethan-Einheit), 1,4 (s, 9H, $C(CH_3)_3$), 1,93 (bs, 15H, $COCCH_3$), 3,4 (m, 2H, $NCH_2$), 4,3 (m, 10H, $OCH_2$), 5,3 (m, 2H, O-CH), 5,6, 6,12 (2m, je 5H, vinyl-H) ppm.

$^{13}$C-NMR ($CDCl_3$, 90 MHz): δ = 18,3, 28,7, 29,4 ($CH_3$), um 30 (C der Dicyclohexylmethan-Einheit), 43,2 ($NCH_2$), 55,9 ($\underline{C}(CH_3)_3$), 62,7 ($OCH_2$), 69,5 (O-CH), 126,2 (= $CH_2$), 135,8 (=$\underline{C}CH_3$), 155,0 (O-CO-NH), 158,5 (N-CO-N), 166,7 (O-$\underline{C}$O-C) ppm.

Beispiel 6

Die Monomermischung gemäß Beispiel 3 bestehend aus 70 Gew.-% des Adduktes aus M-Triisocyanat, GDMA und BAEMA und 30 Gew.-% HDMA wird versetzt mit 0,2 Gew.-% Campherchinon und 0,5 Gew.-% 4-N,N-Dimethylaminobenzolsulfonsäurebisallylamid und unter Lichtausschluß zu einer aktivierten Monomermischung verarbeitet. Diese härtet durch sichtbares Licht bei einer Belichtungsdauer von 60s zu einem Kunststoff hoher mechanischer Stabilität aus und ist als Verschlußmaterial im Dentalbereich (Sealer, Liner, Zahnlack) verwendbar.

Zur Herstellung einer Zahnfüllmasse werden 25 Gewichtsteile der aktivierten Monomermischung und 75 Gewichtsteile einer von mit 3-Methacryloyloxypropyltrimethoxysilan silanisierten Mischung aus pyrogener Kieselsäure und gemahlenen Quarzgläsern in einem handelsüblichen Kneter bei Raumtempertur zu einer Paste verarbeitet. Ein nach DIN 13 922 mit einer handelsüblichen Dentallampe (Translux$^R$) ausgehärteter Probekörper, der aus dieser Paste hergestellt wurde, zeigt neben einem hohen Biegemodul und einer hohen Biegefestigkeit insbesondere eine gute Abrasionsbeständigkeit.

**Patentansprüche**

**Patentansprüche für forgende Vertragsstaaten : AT, BE, CH, DE, DK, FR, GB, GR, IT, LI, LU, NL, SE**

1. (Meth)-Acrylsäure-Derivate von Triisocyanaten der Formel

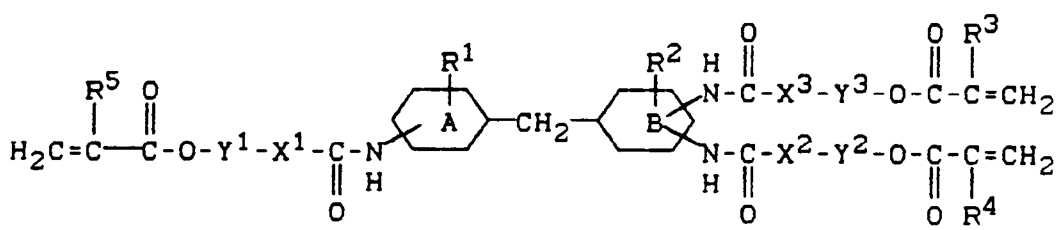

(I)

in der

$R^1$ und $R^2$ gleich oder verschieden sind und für Wasserstoff oder einen Niederalkylrest stehen,

$R^3$, $R^4$ und $R^5$ gleich oder verschieden sind und Wasserstoff oder Methyl bedeuten,

$Y^1$ bis $Y^3$ gleich oder verschieden sind und zweiwertige geradkettige oder verzweigte Kohlenwasserstoffreste mit 2 bis 15 Kohlenstoffatomen, die gegebenenfalls 1 bis 3 Sauerstoffbrücken enthalten können und gegebenenfalls durch 1 bis 4 zusätzliche (Meth)-Acryloyl-oxy-Reste substituiert sein können, bedeuten,

$X^1$ bis $X^3$ gleich oder verschieden sind und $-NR^1-$ oder -O- bedeuten, wobei mindestens einer der Reste $X^1$ bis $X^3$ $-NR^1-$ bedeutet und $R^1$ die oben beschriebene Bedeutung hat und die Ringe

A und B gleich oder verschieden sind und aromatisch oder gesättigt sein können.

2. (Meth)-Acrylsäure-Derivate von Triisocyanaten der Formel I nach Anspruch 1,

worin

$R^1$ für Wasserstoff steht,

$R^2$ für Wasserstoff oder einen Alkylrest mit 1 bis 4 Kohlenstoffatomen steht,

$R^3$, $R^4$ und $R^5$ gleich oder verschieden sind und Wasserstoff oder Methyl bedeuten,

$Y^1$ bis $Y^3$ gleich oder verschieden sind und zweiwertige geradkettige oder verzweigte aliphatische Kohlenwasserstoffreste mit 2 bis 10 Kohlenstoffatomen, die gegebenenfalls 1 oder 2 Sauerstoffbrücken enthalten können und gegebenenfalls durch 1 oder 2 zusätzliche (Meth)-Acryloyloxy-Reste substituiert sein können, bedeuten,

$X^1$ für -O- steht,

$X^2$ und $X^3$ die Bedeutung von -$NR^1$- haben,

der Ring B aromatisch oder gesättigt ist und der Ring A gesättigt ist.

**3.** (Meth)-Acrylsäure-Derivate von Triisocyanaten der Formel I nach den Ansprüchen 1 und 2,

worin

$R^1$ für Wasserstoff steht,

$R^2$ für Wasserstoff oder Methyl steht,

$R^3$, $R^4$ und $R^5$ gleich oder verschieden sind und Wasserstoff oder Methyl bedeuten,

$Y^1$ bis $Y^3$ gleich oder verschieden sind und zweiwertige geradkettige oder verzweigte aliphatische Kohlenwasserstoffreste mit 2 bis 10 Kohlenstoffatomen, die gegebenenfalls 1 oder 2 Sauerstoffbrücken enthalten können und gegebenenfalls durch 1 oder 2 zusätzliche (Meth)-Acryloyloxy-Reste substituiert sein können, bedeuten,

$X^1$ und $X^2$ für -O- stehen,

$X^3$ die Bedeutung von $NR^1$ hat, und die Ringe A und B gesättigt sind.

**4.** Verfahren zur Herstellung der (Meth)-Acrylsäure-Derivate nach den Ansprüchen 1 bis 3, dadurch gekennzeichnet, daß man ein Triisocyanat der Formel II

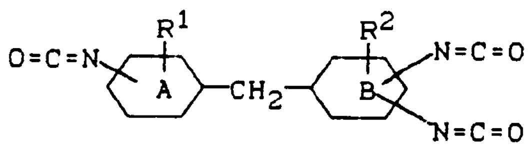

(II)

in der

$R^1$ und $R^2$ gleich oder verschieden sind und für Wasserstoff oder einen Niederalkylrest stehen, und die Ringe A und B gleich oder verschieden sind und aromatisch oder gesättigt sein können,

mit Aminoalkyl-(Meth)-Acrylsäureestern der Formel

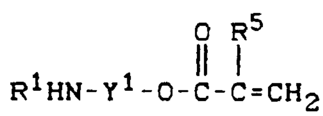

und/oder

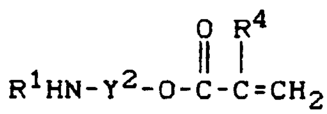

und/oder

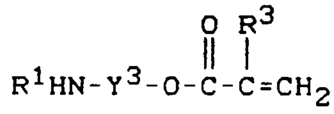

und gegebenenfalls mit Hydroxalkyl-(Meth)-Acrylsäureester der Formel

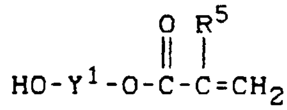

und/oder

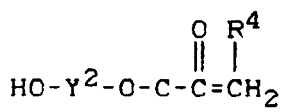

und/oder

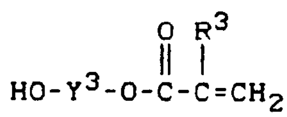

in denen

$R^3$, $R^4$ und $R^5$ gleich oder verschieden sind und Wasserstoff oder Methyl bedeuten und

$Y^1$ und $Y^3$ gleich oder verschieden sind und zweiwertige geradkettige oder verzweigte aliphatische Kohlenwasserstoffreste mit 2 bis 15 Kohlenstoffatomen, die gegebenenfalls 1 bis 3 Sauerstoffbrücken enthalten können und gegebenenfalls durch 1 bis 4 zusätzliche (Meth)-Acryloyloxy-Reste substituiert sein können, bedeuten und

$R^1$ die oben beschriebene Bedeutung hat, umsetzt.

5. Polymerisat aus (Meth)-Acrylsäure-Derivaten von Triisocyanaten der Formel I

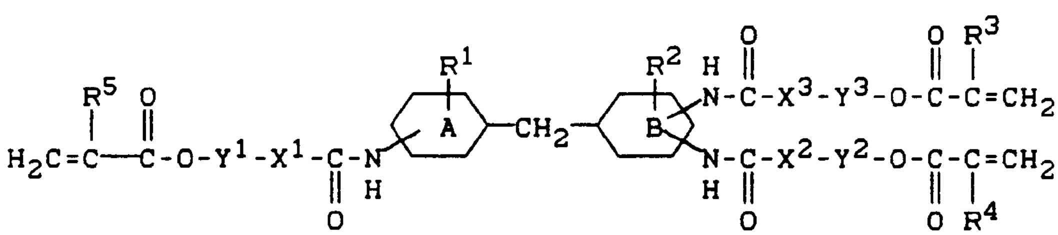

(I)

in der

$R^1$ und $R^2$ gleich oder verschieden sind und für Wasserstoff oder einen Niederalkylrest stehen,

$R^3$, $R^4$ und $R^5$ gleich oder verschieden sind und Wasserstoff oder Methyl bedeuten,

$Y^1$ bis $Y^3$ gleich oder verschieden sind und zweiwertige geradkettige oder verzweigte Kohlenwasserstoffreste mit 2 bis 15 Kohlenstoffatomen, die gegebenenfalls 1 bis 3 Sauerstoffbrücken enthalten können und gegebenenfalls durch 1 bis 4 zusätzliche (Meth)-Acryloyloxy-Reste substituiert sein können, be-

deuten,

$X^1$ und $X^3$ gleich oder verschieden sind und $-NR^1$oder -O- bedeuten, wobei mindestens einer der Reste $X^1$ bis $X^3$ $-NR^1-$ bedeutet und $R^1$ die oben beschriebene Bedetung hat und die Ringe A und B gleich oder verschieden sind und aromatisch oder gesättigt sein können.

6. Verwendung von (Meth)-Acrylsäure-Derivaten von Triisocyanaten der Formel I

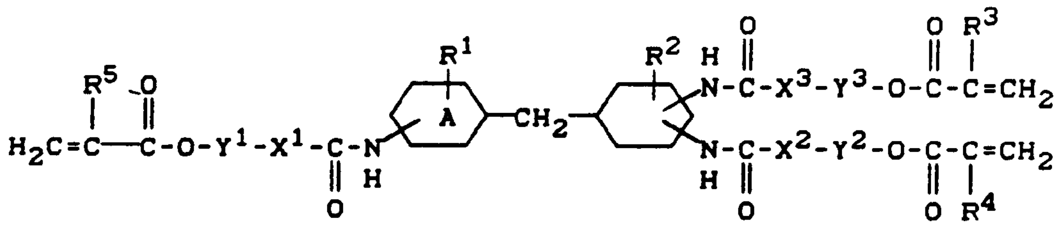

(I)

in der

$R^1$ und $R^2$ gleich oder verschieden sind und für Wasserstoff oder einen Niederalkylrest stehen,

$R^3$, $R^4$ und $R^5$ gleich oder verschieden sind und Wasserstoff oder Methyl bedeuten,

$Y^1$ bis $Y^3$ gleich oder verschieden sind und zweiwertige geradkettige oder verzweigte Kohlenwasserstoffreste mit 2 bis 15 Kohlenstoffatomen, die gegebenenfalls 1 bis 3 Sauerstoffbrücken enthalten können und gegebenenfalls durch 1 bis 4 zusätzliche (Meth)-Acryloylox-Reste substituiert sein können,

$X^1$ bis $X^3$ gleich oder verschieden sind und $-NR^1$oder -O- bedeuten, wobei mindestens einer der Reste $X^1$ bis $X^3$ $-NR^1-$ bedeutet und $R^1$ die oben beschriebene Bedeutung hat und die Ringe A und B gleich oder verschieden sind und aromatisch oder gesättigt sein können,

im Dentalbereich.

7. Verwendung nach Anspruch 6, dadurch gekennzeichnet, daß die (Meth)-Acrylsäure-Derivate von Triisocyanaten der Formel I in Zahnfüllmassen eingesetzt werden.

8. Verwendung nach Anspruch 6, dadurch gekennzeichnet, daß die (Meth)-Acrylsäure-Derivate von Triisocyanaten der Formel I in Beschichtungsmitteln für Zähne eingesetzt werden.

9. Verwendung nach Anspruch 6, dadurch gekennzeichnet, daß die (Meth)-Acrylsäure-Derivate von Triisocyanaten der Formel I für die Herstellung von Kunststoffzähnen eingesetzt werden.

10. Dentalmaterialien, dadurch gekennzeichnet, daß sie (Meth)-Acrylsäure-Derivate von Triisocyanaten der Formel I

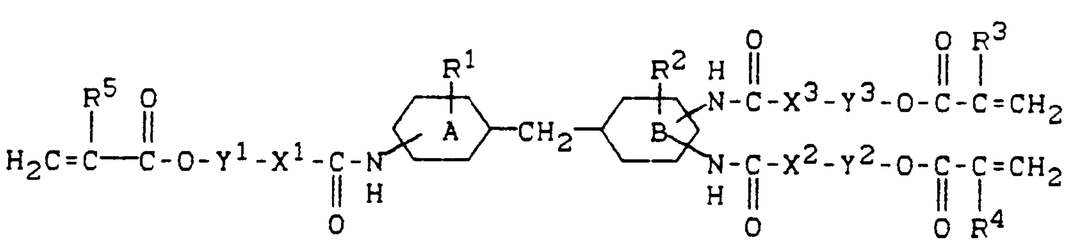

(I)

in der

$R^1$ und $R^2$ gleich oder verschieden sind und für Wasserstoff oder einen Niederalkylrest stehen,

$R^3$, $R^4$ und $R^5$ gleich oder verschieden sind und Wasserstoff oder Methyl bedeuten,

$Y^1$ bis $Y^3$ gleich oder verschieden sind und zweiwertige geradkettige oder verzweigte aliphatische Kohlenwasserstoffreste mit 2 bis 15 Kohlenstoffatomen, die gegebenenfalls 1 bis 3 Sauerstoffbrücken enthalten können und gegebenenfalls durch 1 bis 4 zusätzliche (Meth)-Acryloyloxy-Reste substituiert sein können,

$X^1$ bis $X^3$ gleich oder verschieden sind und $-NR^1$oder -O- bedeuten, wobei mindestens einer der Reste $X^1$ bis $X^3$ $-NR^1$- bedeutet und $R^1$ die oben beschriebene Bedeutung hat und die Ringe A und B gleich oder verschieden sind und aromatisch oder gesättigt sein können, enthalten.

**Patentanspruch für folgenden Vertragstaat : ES**

1. Verfahren zur Herstellung von (Meth)-Acrylsäure-Derivaten von Triisocyanaten der Formel

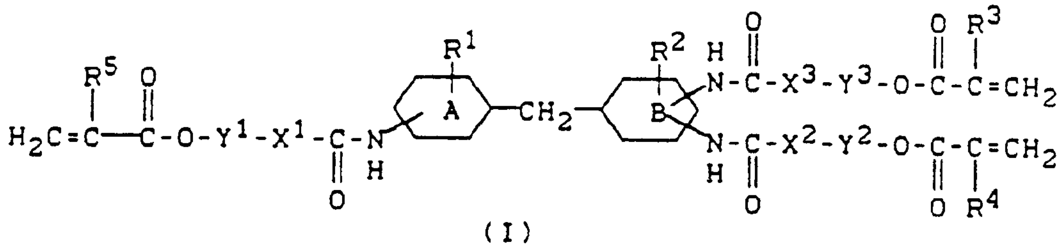

(I)

in der

$R^1$ und $R^2$ gleich oder verschieden sind und für Wasserstoff oder einen Niederalkylrest stehen,

$R^3$, $R^4$ und $R^5$ gleich oder verschieden sind und Wasserstoff oder Methyl bedeuten,

$Y^1$ bis $Y^3$ gleich oder verschieden sind und zweiwertige geradkettige oder verzweigte Kohlenwasserstoffreste mit 2 bis 15 Kohlenstoffatomen, die gegebenenfalls 1 bis 3 Sauerstoffbrücken enthalten können und gegebenenfalls durch 1 bis 4 zusätzliche (Meth)-Acryloyl-oxy-Reste substituiert sein können, bedeuten,

$X^1$ bis $X^3$ gleich oder verschieden sind und $-NR^1$- oder -O- bedeuten, wobei mindestens einer der Reste $X^1$ bis $X^3$ $-NR^1$- bedeutet und $R^1$ die oben beschriebene Bedeutung hat und die Ringe A und B gleich oder verschieden sind und aromatisch oder gesättigt sein können.

dadurch gekennzeichnet, daß man ein Triisocyanat der Formel II

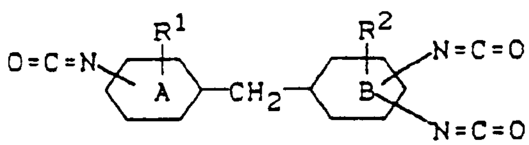

(II)

in der

$R^1$ und $R^2$ gleich oder verschieden sind und für Wasserstoff oder einen Niederalkylrest stehen, und die Ringe A und B gleich oder verschieden sind und aromatisch oder gesättigt sein können,

mit Aminoalkyl-(Meth)-Acrylsäureestern der Formel

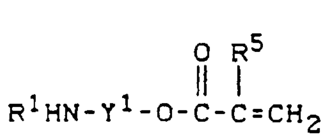

und/oder

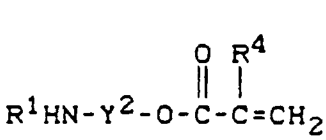

und/oder

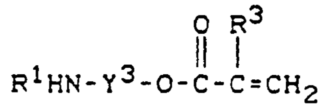

und gegebenenfalls mit Hydroxalkyl-(Meth)-Acrylsäureester der Formel

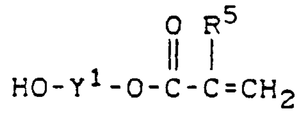

und/oder

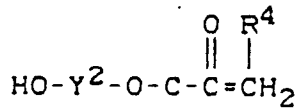

und/oder

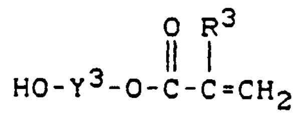

in denen

$R^3$, $R^4$ und $R^5$ gleich oder verschieden sind und Wasserstoff oder Methyl bedeuten und

$Y^1$ und $Y^3$ gleich oder verschieden sind und zweiwertige geradkettige oder verzweigte aliphatische Kohlenwasserstoffreste mit 2 bis 15 Kohlenstoffatomen, die gegebenenfalls 1 bis 3 Sauerstoffbrücken enthalten können und gegebenenfalls durch 1 bis 4 zusätzliche (Meth)-Acryloyloxy-Reste substituiert sein können, bedeuten und

$R^1$ die oben beschriebene Bedeutung hat, umsetzt.

## Claims

### Claims for the following Contracting States : AT, BE, CH, DE, DK, FR, GB, GR, IT, LI, LU, NL, SE

**1.** (Meth)acrylic acid derivatives of triisocyanates of the formula

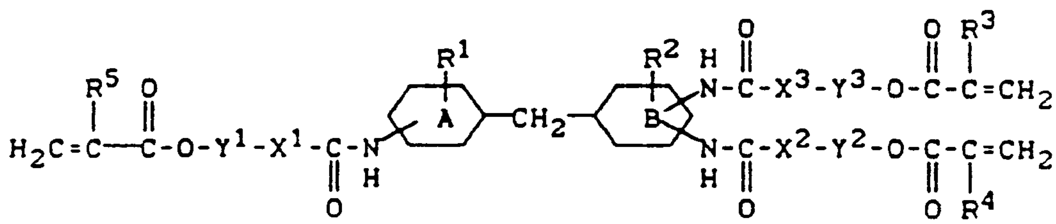

(I)

wherein

$R^1$ and $R^2$ are identical or different and represent hydrogen or a lower alkyl radical,

$R^3$, $R^4$ and $R^5$ are identical or different and denote hydrogen or methyl,

$Y^1$ to $Y^3$ are identical or different and denote divalent straight-chain or branched hydrocarbon radicals of 2 to 15 carbon atoms which may optionally comprise 1 to 3 oxygen bridges and which may be unsubstituted or substituted by 1 to 4 additional (meth)acryloyloxy radicals,

$X^1$ to $X^3$ are identical or different and denote $-NR^1-$ or -O-, at least one of the radicals $X^1$ to $X^3$ denoting $-NR^1-$, $R^1$ having the meaning defined above, and the rings

A and B are identical or different and may be aromatic or saturated.

2. (Meth)acrylic acid derivatives of triisocyanates of the formula I according to Claim 1,
wherein
$R^1$ represents hydrogen,
$R^2$ represents hydrogen or an alkyl radical of 1 to 4 carbon atoms,
$R^3$, $R^4$ and $R^5$ are identical or different and denote hydrogen or methyl,
$Y^1$ to $Y^3$ are identical or different and denote divalent straight-chain or branched aliphatic hydrocarbon radicals of 2 to 10 carbon atoms which may optionally comprise 1 or 2 oxygen bridges and which may be unsubstituted or substituted by 1 or 2 additional (meth)acryloyloxy radicals,
$X^1$ represents -O-,
$X^2$ and $X^3$ denote $-NR^1-$,
the ring B is aromatic or saturated and the ring A is saturated.

3. (Meth)acrylic acid derivatives of triisocyanates of the formula I according to Claims 1 and 2,
wherein
$R^1$ represents hydrogen,
$R^2$ represents hydrogen or methyl,
$R^3$, $R^4$ and $R^5$ are identical or different and denote hydrogen or methyl,
$Y^1$ to $Y^3$ are identical or different and denote divalent straight-chain or branched aliphatic hydrocarbon radicals of 2 to 10 carbon atoms which may optionally comprise 1 or 2 oxygen bridges and which may be unsubstituted or substituted by 1 or 2 additional (meth)acryloyloxy radicals,
$X^1$ and $X^2$ represent -O-,
$X^3$ denotes $NR^1$, and the rings
A and B are saturated.

4. Process for the preparation of the (meth)acrylic acid derivatives according to Claims 1 to 3, characterized in that a triisocyanate of the formula II

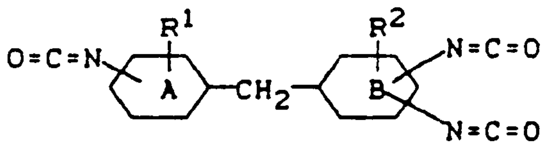

(II)

wherein
$R^1$ and $R^2$ are identical or different and represent hydrogen or a lower alkyl radical, and the rings A and B are identical or different and may be aromatic or saturated,
is reacted with aminoalkyl (meth)acrylates of the formula

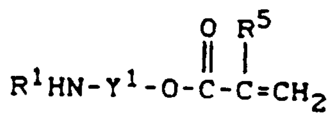

and/or

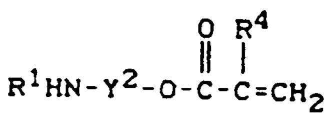

and/or

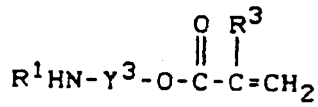

and optionally with hydroxyalkyl (meth)acrylates of the formula

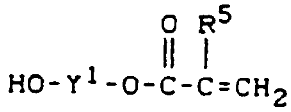

and/or

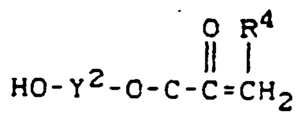

and/or

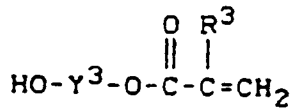

wherein

$R^3$, $R^4$ and $R^5$ are identical or different and denote hydrogen or methyl and

$Y^1$ to $Y^3$ are identical or different and denote divalent straight-chain or branched aliphatic hydrocarbon radicals of 2 to 15 carbon atoms which may optionally comprise 1 or 3 oxygen bridges and which may be unsubstituted or substituted by 1 to 4 (meth)acryloyloxy radicals, and

$R^1$ has the meaning defined above.

5. Polymer from (meth)acrylic acid derivatives of triisocyanates of the formula I

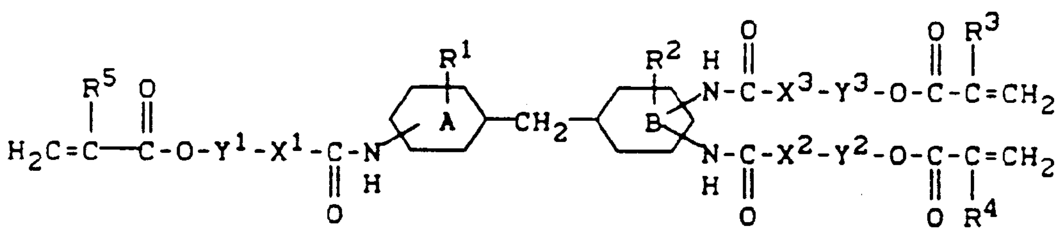

(I)

wherein

$R^1$ and $R^2$ are identical or different and represent hydrogen or a lower alkyl radical,

$R^3$, $R^4$ and $R^5$ are identical or different and denote hydrogen or methyl,

$Y^1$ to $Y^3$ are identical or different and denote divalent straight-chain or branched hydrocarbon radicals of 2 to 15 carbon atoms which may optionally comprise 1 to 3 oxygen bridges and which may be unsubstituted or substituted by 1 to 4 additional (meth)acryloyloxy radicals,

$X^1$ and $X^3$ are identical or different and denote -$NR^1$- or -O-, at least one of the radicals $X^1$ to $X^3$ denoting -$NR^1$- and $R^1$ having the meaning defined above, and the rings

A and B are identical or different and may be aromatic or saturated.

6. Use of (meth)acrylic acid derivatives of triisocyanates of the formula I

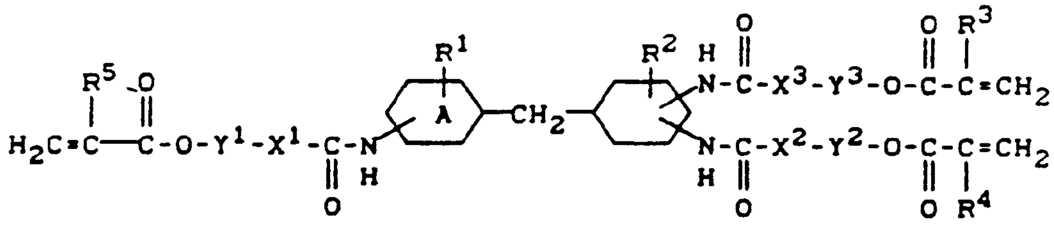

(I)

wherein

$R^1$ and $R^2$ are identical or different and represent hydrogen or a lower alkyl radical,

$R^3$, $R^4$ and $R^5$ are identical or different and denote hydrogen or methyl,

$Y^1$ to $Y^3$ are identical or different and denote divalent straight-chain or branched hydrocarbon radicals of 2 to 15 carbon atoms which may optionally comprise 1 to 3 oxygen bridges and which may be unsubstituted or substituted by 1 to 4 additional (meth)acryloyloxy radicals,

$X^1$ to $X^3$ are identical or different and denote $-NR^1-$ or -O-, at least one of the radicals $X^1$ to $X^3$ denoting $-NR^1-$ and $R^1$ having the meaning defined above, and the rings

A and B are identical or different and may be aromatic or saturated,

in dentistry.

7. Use according to Claim 6, characterized in that the (meth)acrylic acid derivatives of triisocyanates of the formula I are used in dental filling compounds.

8. Use according to Claim 6, characterized in that the (meth)acrylic acid derivatives of triisocyanates of the formula I are used in dental coatings.

9. Use according to Claim 6, characterized in that the (meth)acrylic acid derivatives of triisocyanates of the formula I are used for the preparation of artificial teeth.

10. Dental materials, characterized in that they contain (meth)acrylic acid derivatives of triisocyanates of the formula I

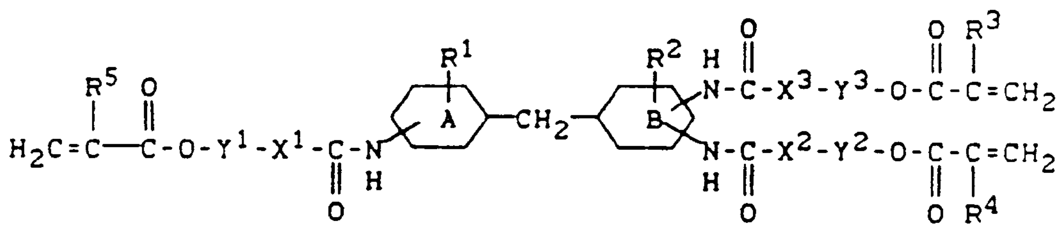

(I)

wherein

$R^1$ and $R^2$ are identical or different and represent hydrogen or a lower alkyl radical,

$R^3$, $R^4$ and $R^5$ are identical or different and denote hydrogen or methyl,

$Y^1$ to $Y^3$ are identical or different and denote divalent straight-chain or branched aliphatic hydrocarbon radicals of 2 to 15 carbon atoms which may optionally comprise 1 to 3 oxygen bridges and which may be unsubstituted or substituted by 1 to 4 additional (meth)acryloyloxy radicals,

$X^1$ to $X^3$ are identical or different and denote $-NR^1-$ or -O-, at least one of the radicals $X^1$ to $X^3$ denoting $-NR^1-$ and $R^1$ having the meaning defined above, and the rings

A and B are identical or different and may be aromatic or saturated.

**Claim for the following Contracting State : ES**

1. Process for the preparation of (meth)acrylic acid derivatives of triisocyanates of the formula

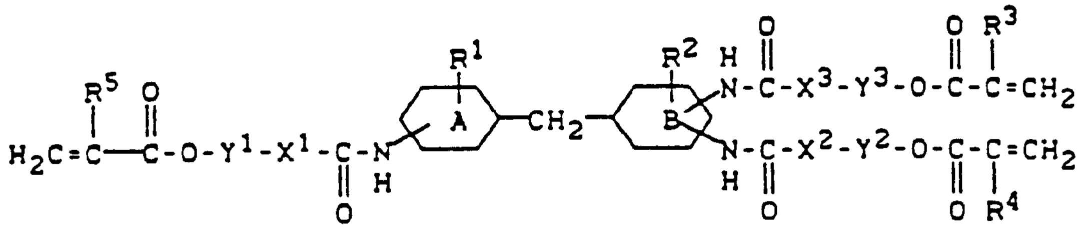

(I)

wherein

$R^1$ and $R^2$ are identical or different and represent hydrogen or a lower alkyl radical,

$R^3$, $R^4$ and $R^5$ are identical or different and denote hydrogen or methyl,

$Y^1$ to $Y^3$ are identical or different and denote divalent straight-chain or branched hydrocarbon radicals of 2 to 15 carbon atoms which may optionally comprise 1 to 3 oxygen bridges and which may be unsubstituted or substituted by 1 to 4 additional (meth)acryloyloxy radicals,

$X^1$ to $X^3$ are identical or different and denote $-NR^1-$ or -O-, at least one of the radicals $X^1$ to $X^3$ denoting $-NR^1-$, $R^1$ having the meaning defined above, and the rings

A and B are identical or different and may be aromatic or saturated,

characterized in that a triisocyanate of the formula II

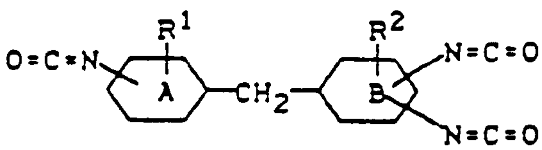

(II)

wherein

$R^1$ and $R^2$ are identical or different and represent hydrogen or a lower alkyl radical, and the rings A and B are identical or different and may be aromatic or saturated,

is reacted with aminoalkyl (meth)acrylates of the formula

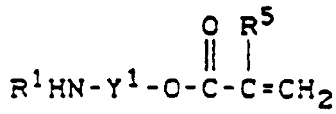

and/or

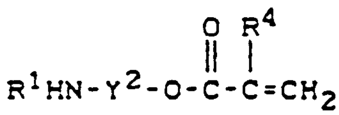

and/or

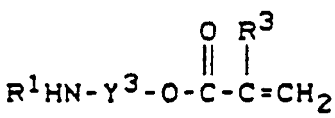

and optionally with hydroxyalkyl (meth)acrylates of the formula

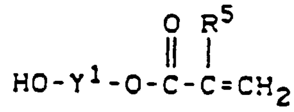

and/or

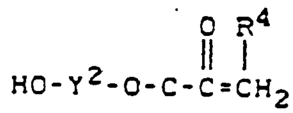

and/or

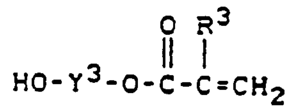

wherein

$R^3$, $R^4$ and $R^5$ are identical or different and denote hydrogen or methyl and

$Y^1$ to $Y^3$ are identical or different and denote divalent straight-chain or branched aliphatic hydrocarbon radicals of 2 to 15 carbon atoms which may optionally comprise 1 or 3 oxygen bridges and which may be unsubstituted or substituted by 1 to 4 (meth)acryloyloxy radicals, and

$R^1$ has the meaning defined above.

## Revendications

### Revendications pour les Etats contractants suivants : AT, BE, CH, DE, DK, FR, GB, GR, IT, LI, LU, NL, SE

**1.** Dérivés d'acide (méth)acrylique de triisocyanates de formule

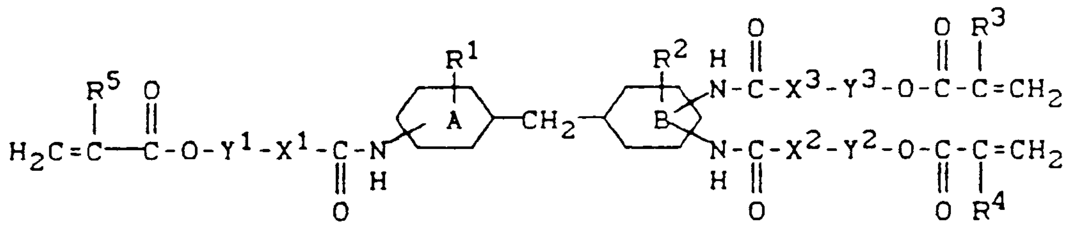

(I)

dans laquelle

$R^1$ et $R^2$ sont identiques ou différents et représentent l'hydrogène ou un reste alkyle inférieur,

$R^3$, $R^4$ et $R^5$ sont identiques ou différents et représentent l'hydrogène ou un reste méthyle,

$Y^1$ à $Y^3$ sont identiques ou différents et représentent des restes hydrocarbonés divalents à chaîne droite ou ramifiée ayant 2 à 15 atomes de carbone, qui peuvent éventuellement contenir 1 à 3 ponts oxygène et qui peuvent être substitués le cas échéant par 1 à 4 restes (méth)acryloyloxy additionnels,

$X^1$ à $X^3$ sont identiques ou différents et représentent un groupe $-NR^1-$ ou -O-, l'un au moins des restes $X^1$ à $X^3$ étant un groupe $-NR^1-$ et $R^1$ ayant la définition indiquée ci-dessus et les noyaux

A et B sont identiques ou différents et peuvent être aromatiques ou saturés.

**2.** Dérivés d'acide (méth)acrylique de triisocyanates de formule I suivant la revendication 1, dans lesquels

$R^1$ représente l'hydrogène,

$R^2$ est l'hydrogène ou un reste alkyle ayant 1 à 4 atomes de carbone,

$R^3$, $R^4$ et $R^5$ sont identiques ou différents et représentent l'hydrogène ou le reste méthyle,

$Y^1$ à $Y^3$ sont identiques ou différents et représentent des restes divalents d'hydrocarbures aromatiques à chaîne droite ou ramifiée ayant 2 à 10 atomes de carbone, qui peuvent éventuellement contenir 1 ou 2 ponts oxygène et qui peuvent être substitués le cas échéant par 1 ou 2 restes (méth)acryloyloxy additionnels,

$X^1$ représente -O-,

$X^2$ et $X^3$ ont la définition de $-NR^1-$,

le noyau B est aromatique ou saturé et le noyau A est saturé.

3. Dérivés d'acide (méth)acrylique de triisocyanates de formule I suivant les revendications 1 et 2, dans lesquels

$R^1$ représente l'hydrogène

$R^2$ est l'hydrogène ou le reste méthyle,

$R^3$, $R^4$ et $R^5$ sont identiques ou différents et représentent l'hydrogène ou le reste méthyle,

$Y^1$ à $Y^3$ sont identiques ou différents et sont des restes divalents d'hydrocarbures aliphatiques à chaîne droite ou ramifiée ayant 2 à 10 atomes de carbone, qui peuvent contenir, le cas échéant, 1 ou 2 ponts oxygène et qui peuvent être substitués éventuellement par 1 ou 2 restes (méth)acryloyloxy additionnels,

$X^1$ et $X^2$ représente -O-,

$X^3$ a la définition de $NR^1$, et les noyaux A et B sont saturés.

4. Procédé de production des dérivés d'acide (méth)acrylique suivant les revendications 1 à 3, caractérisé en ce qu'on fait réagir un triisocyanate de formule II

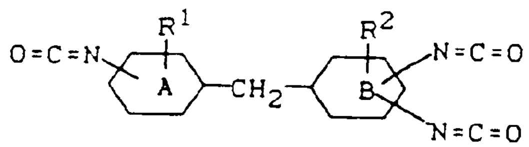

(II)

dans laquelle

$R^1$ et $R^2$ sont identiques ou différents et représentent l'hydrogène ou un reste alkyle inférieur et les noyaux A et B sont identiques ou différents et peuvent être aromatiques ou saturés,

avec des esters amino-alkyliques d'acide (méth)acrylique de formules

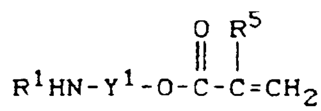

et/ou

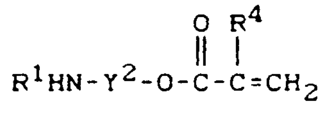

et/ou

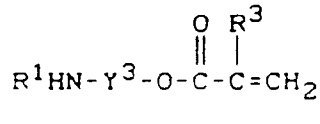

et, le cas échéant, avec des esters hydroxyalkyliques d'acide (méth)acrylique de formules

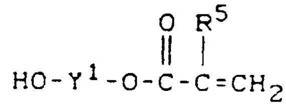

et/ou

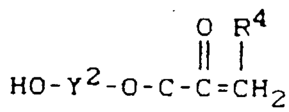

et/ou

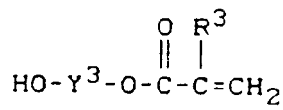

formules dans lesquelles

$R^3$, $R^4$ et $R^5$ sont identiques ou différents et désignent l'hydrogène ou le reste méthyle et

$Y^1$ à $Y^3$ sont identiques ou différents et représentent des restes divalents d'hydrocarbures aliphatiques à chaîne droite ou ramifiée ayant 2 à 15 atomes de carbone, qui peuvent contenir, le cas échéant, 1 à 3 ponts oxygène et qui peuvent être éventuellement substitués par 1 à 4 restes (méth)acryloyloxy additionnels, et

$R^1$ a la définition donnée ci-dessus.

**5.** Produit de polymérisation de dérivés d'acide (méth)acrylique de triisocyanates de formule I

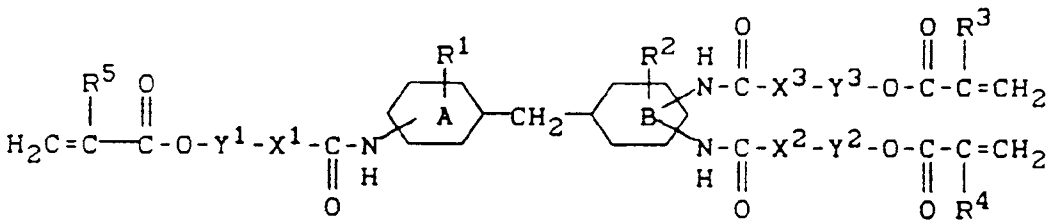

(I)

dans laquelle

$R^1$ et $R^2$ sont identiques ou différents et représentent l'hydrogène ou un reste alkyle inférieur,

$R^3$, $R^4$ et $R^5$ sont identiques ou différents et représentent l'hydrogène ou un reste méthyle,

$Y^1$ à $Y^3$ sont identiques ou différents et représentent des restes hydrocarbonés divalents à chaîne droite ou ramifiée ayant 2 à 15 atomes de carbone, qui peuvent éventuellement contenir 1 à 3 ponts oxygène et qui peuvent être substitués, le cas échéant, par 1 à 4 restes (méth)acryloyloxy additionnels,

$X^1$ à $X^3$ sont identiques ou différents et représentent un groupe $-NR^1-$ ou -O-, l'un au moins des restes $X^1$ à $X^3$ étant un groupe $-NR^1-$ et $R^1$ ayant la définition indiquée ci-dessus et les noyaux

A et B sont identiques ou différents et peuvent être aromatiques ou saturés.

**6.** Utilisation de dérivés d'acide (méth)acrylique de triisocyanates de formule I

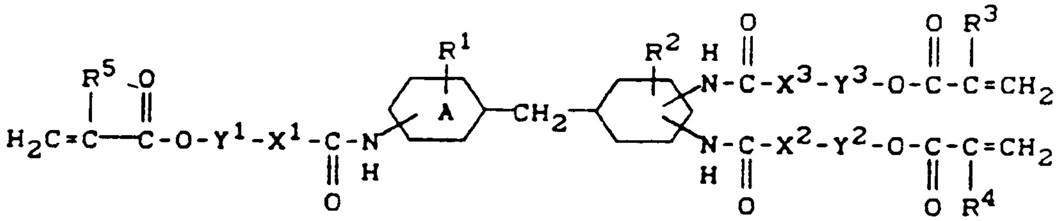

(I)

dans laquelle

$R^1$ et $R^2$ sont identiques ou différents et représentent l'hydrogène ou un reste alkyle inférieur,

$R^3$, $R^4$ et $R^5$ sont identiques ou différents et représentent l'hydrogène ou un reste méthyle,

$Y^1$ à $Y^3$ sont identiques ou différents et représentent des restes hydrocarbonés divalents à chaîne droite ou ramifiée ayant 2 à 15 atomes de carbone, qui peuvent éventuellement contenir 1 à 3 ponts oxygène et qui peuvent être substitués, le cas échéant, par 1 à 4 restes (méth)acryloyloxy additionnels,

$X^1$ à $X^3$ sont identiques ou différents et représentent un groupe $-NR^1-$ ou -O-, l'un au moins des restes $X^1$ à $X^3$ étant un groupe $-NR^1-$ et $R^1$ ayant la définition indiquée ci-dessus et les noyaux

A et B sont identiques ou différents et peuvent être aromatiques ou saturés,

dans le domaine de l'art dentaire.

**7.** Utilisation suivant la revendication 6, caractérisée en ce que les dérivés d'acide (méth)acrylique de triisocyanates de formule I sont utilisés dans des matières d'obturation dentaire.

**8.** Utilisation suivant la revendication 6, caractérisée en ce que les dérivés d'acide (méth)acrylique de triisocyanates de formule I sont utilisés dans des compositions de revêtement à usage dentaire.

**9.** Utilisation suivant la revendication 6, caractérisée en ce que les dérivés d'acide (méth)acrylique de triisocyanates de formule I sont utilisés pour la production de dents en matière plastique.

**10.** Matériaux dentaires, caractérisés en ce qu'ils contiennent des dérivés d'acide (méth)acrylique de triisocyanates de formule I

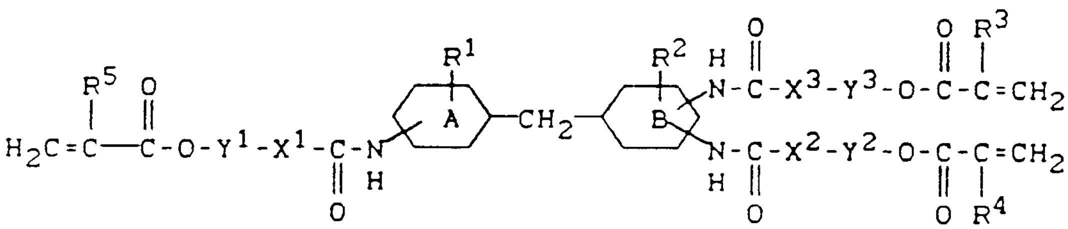

(I)

dans laquelle

$R^1$ et $R^2$ sont identiques ou différents et représentent l'hydrogène ou un reste alkyle inférieur,

$R^3$, $R^4$ et $R^5$ sont identiques ou différents et représentent l'hydrogène ou un reste méthyle,

$Y^1$ à $Y^3$ sont identiques ou différents et représentent des restes hydrocarbonés divalents à chaîne droite ou ramifiée ayant 2 à 15 atomes de carbone, qui peuvent éventuellement contenir 1 à 3 ponts oxygène et qui peuvent être substitués, le cas échéant, par 1 à 4 restes (méth)acryloyloxy additionnels,

$X^1$ à $X^3$ sont identiques ou différents et représentent un groupe $-NR^1-$ ou -O-, l'un au moins des restes $X^1$ à $X^3$ étant un groupe $-NR^1-$ et $R^1$ ayant la définition indiquée ci-dessus et les noyaux

A et B sont identiques ou différents et peuvent être aromatiques ou saturés.

**Revendication pour l'Etat contractant suivant : ES**

**1.** Procédé de production de dérivés d'acide (méth)acrylique de triisocyanates de formule

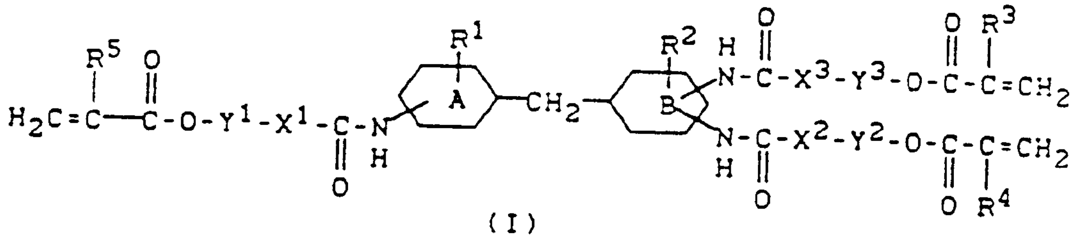

(I)

dans laquelle

$R^1$ et $R^2$ sont identiques ou différents et représentent l'hydrogène ou un reste alkyle inférieur,

$R^3$, $R^4$ et $R^5$ sont identiques ou différents et représentent l'hydrogène ou un reste méthyle,

$Y^1$ à $Y^3$ sont identiques ou différents et représentent des restes hydrocarbonés divalents à chaîne droite ou ramifiée ayant 2 à 15 atomes de carbone, qui peuvent éventuellement contenir 1 à 3 ponts oxygène et qui peuvent être substitués, le cas échéant, par 1 à 4 restes (méth)acryloyloxy additionnels,

$X^1$ à $X^3$ sont identiques ou différents et représentent un groupe $-NR^1-$ ou -O-, l'un au moins des restes $X^1$ à $X^3$ étant un groupe $-NR^1-$ et $R^1$ ayant la définition indiquée ci-dessus et les noyaux

A et B sont identiques ou différents et peuvent être aromatiques ou saturés,

caractérisé en ce qu'on fait réagir un triisocyanate de formule II

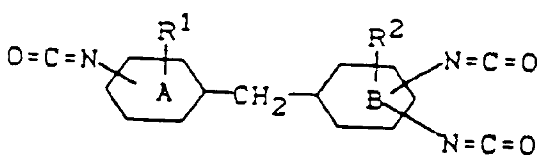

(II)

dans laquelle

$R^1$ et $R^2$ sont identiques ou différents et représentent l'hydrogène ou un reste alkyle inférieur et les noyaux A et B sont identiques ou différents et peuvent être aromatiques ou saturés,

avec des esters amino-alkyliques d'acide (méth)acrylique de formules

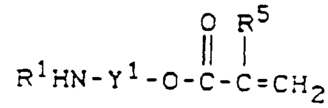

et/ou

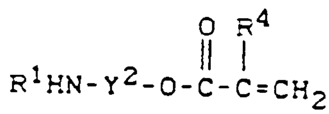

et/ou

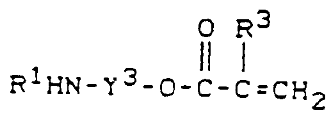

et, le cas échéant, avec des esters hydroxyalkyliques d'acide (méth)acrylique de formules

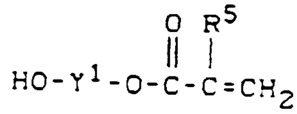

et/ou

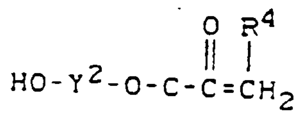

et/ou

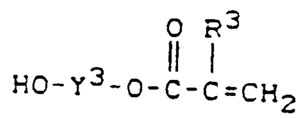

formules dans lesquelles

$R^3$, $R^4$ et $R^5$ sont identiques ou différents et désignent l'hydrogène ou le reste méthyle et

$Y^1$ à $Y^3$ sont identiques ou différents et représentent des restes divalents d'hydrocarbures aliphatiques à chaîne droite ou ramifiée ayant 2 à 15 atomes de carbone, qui peuvent contenir, le cas échéant, 1 à 3 ponts oxygène et qui peuvent être éventuellement substitués par 1 à 4 restes (méth)acryloyloxy additionnels, et

$R^1$ a la définition donnée ci-dessus.